# EUROPEAN PATENT SPECIFICATION

(11) **EP 3 039 968 B1**
(45) Date of publication and mention of the grant of the patent: **28.02.2018**
(21) Application number: 15199684.0
(22) Date of filing: 11.12.2015
(51) Int. Cl.: A23K 10/12, A23K 10/30, A23K 50/80

(54) **METHOD OF PRODUCING FEED ADDITIVE CONTAINING SURFACTIN**
VERFAHREN ZUR HERSTELLUNG VON FUTTERZUSATZ MIT SURFACTIN
PROCÉDÉ DE PRODUCTION D'UN ADDITIF ALIMENTAIRE CONTENANT UNE SURFACTINE

(30) Priority: 12.12.2014 TW 103143406
(43) Date of publication of application: 06.07.2016
(73) Proprietor: SAFT biotechnology com. LTD, Nanjing (CN); Lu, Jenn-Kan, New Taipei City (TW)
(72) Inventor: Lu, Jenn-Kan, New Taipei City (TW)
(74) Representative: Becker Kurig Straus

(56) References cited:
- DATABASE WPI Week 201318 Thomson Scientific, London, GB; AN 2012-Q82407 XP002757323, & KR 2012 0126746 A (UNIV KONKUK IND COOP CORP) 21 November 2012 (2012-11-21)
- DATABASE WPI Week 201309 Thomson Scientific, London, GB; AN 2012-Q91769 XP002757324, & KR 2012 0129375 A (CHULLANAM-DO) 28 November 2012 (2012-11-28)
- DATABASE WPI Week 201548 Thomson Scientific, London, GB; AN 2015-36454L XP002760545, -& CN 104 509 752 A (FUJIAN ACAD AGRIC SCI CENT LAB) 15 April 2015 (2015-04-15)
- DATABASE WPI Week 201548 Thomson Scientific, London, GB; AN 2015-39221C XP002760546, -& CN 104 543 567 A (FUJIAN ACAD AGRIC SCI CENT LAB) 29 April 2015 (2015-04-29)
- DATABASE BIOSIS [Online] BIOSCIENCES INFORMATION SERVICE, PHILADELPHIA, PA, US; 2016, ZHAI SHAO-WEI ET AL: "Effects of Dietary Surfactin Supplementation on Growth, Digestive Enzyme Activity, and Antioxidant Potential in the Intestine of Growth Retarded Marbled Eel (Anguilla marmaorata) at Elver Stage", XP002760547, Database accession no. PREV201600602559

## Description

### BACKGROUND OF THE INVENTION

### 1. FIELD OF THE INVENTION

The present invention belongs to the field of aquaculture technology and biotechnology in the field of agriculture, involving a method of producing surfactin, a lipopeptide produced by *Bacillus subtilis* and its application in aquafeeds.

### 2. DESCRIPTION OF THE PRIOR ART

The secondary metabolites produced by *Bacillus subtilis* include β-lactams, aminoglycoside, polypetides and small polypeptide, among which lipopeptides and lipoproteins have attracted much attention because of their antibacterial effects and thus are also called Antimicrobial Peptide (AMP).

All AMPs discovered so far can be divided into three families: first, Fengycin which exists as a ring of a peptide chain containing 10 amino acids with 14 to 18 fatty acid side chains attached to its N-terminus has superior anti-fungal activity but shows no apparent effects on yeasts and bacteria (Schneider et al., 1999); the second family is Iturin which comprises a loop of seven α-amino acids coupled with β-amino acids and alkyl chains and the peptide chain is connected with 14-17 branched fatty acid chains, its molecular weight is about 1044 ∼ 1081 kD and its functions are anti-fungal and anti-bacterial effects, it is biodegradable and has high surface activity and low toxicity (Bonmatin, Laprevote, & Peypoux, 2003); the third family is surfactin discussed in this invention, which contains 7 rings of amino acid chains with 13 to 16 side chains of fatty acids. Production of surfactin by *Bacillus subtilis* is usually accompanied by a subtilin and co-existence of the two antimicrobial peptides will increase hemolysis of red blood cells due to the high affinity of the mixed micelles to cells and therefore consequently increase the anti-fungal activity (Feignier, Besson, & Michel, 1995; R Maget-Dana, Thimon, Peypoux, & Ptak, 1992; Sandrin, Peypoux, & Michel, 1990).

In 1968, Arima et al. found a white needle-like crystal in the culture medium of *Bacillus subtilis* and named it surfactin because of its surface activity (Kakinuma, Hori, Isono, Tamura, & Arima, 1969). From previous studies, the surface tension of water can be reduced from 72 mN/m to 27 mN/m by adjusting the concentration of surfactin to 20µM and surfactin is the best biosurfactant identified so far (Arima, Kakinuma, & Tamura, 1968). Surfactin is a ring of lipopeptides containing 7 rings of amino acid chains with 13 to 16 side chains of fatty acids (Kakinuma, Sugino, Isono, Tamura & Arima, 1969). In solutions, the carbon chain of a fatty acid is hydrophobic; on the other hand, the rings of amino acids are the hydrophilic ends, aspartic acid and glutamic acid are both negatively charged and the two negatively charged amino acids will form a clamp which makes surfactin a structure of negatively charged saddle and belongs to the family of anionic biosurfactants (Tsan, Volpon, Besson & Lancelin, 2007). According to the sequences of amino acids they contain, surfactin can be categorized into four types: A, B, C and D. The major difference is the number of fatty acid chains attached to the rings of amino acids varies (Shaligram & Singhal, 2010; Singh & Cameotra, 2004).

Because of the non-specific bioactivity of surfactin, it can destroy the cell membrane of bacteria by reducing surface tension without producing drug-resistance and therefore is considered to have antimicrobial potential and is categorized to the AMP family (Cho, Lee, Cha, Kim & Shin, 2003). Past research has indicated that surfactin can inhibit fungal, mycoplasma, Gram-negative bacteria and Gram-positive bacteria growth (P. Das, Mukherjee & Sen, 2008; Singh & Cameotra, 2004); Heiko et al. reported that surfactin has the antimicrobial effect at the concentration of 12~50µg/ml (Heerklotz & Seelig, 2001); lipopeptide biosurfactants produced by *Bacillus circulans* in the ocean can inhibit the growth of certain multidrug-resistant strain (MDR) and methicillin-resistant Staphylococcus aureus (MRSA) (Pan et al., 2007); moreover; surfactin also has anti-fungal activity and can restore morphological features of cells contaminated by mycoplasma and produces not harm to metabolism and proliferation of the (Vollenbroich, Pauli, Ozel, & Vater, 1997); surfactin was found to cause perforation of mycoplasma membrane by electron microscopy and will lead to death of mycoplasma due to imbalance of outer and inner osmotic pressure (Régine Maget-Dana & Ptak, 1995).

Studies have suggested the anti-viral mechanism of surfactin is caused by structural breakdown of viral envelope and protein coat due to physical and chemical interactions between the lipid envelope of a virus and surfactin as well as inhibition of replication of viral nucleic acid (Vollenbroich, Ozel, Vater, Kamp, & Pauli, 1997). Past studies have pointed out that surfactin is capable of inhibiting the growth of several viruses, including Human Immunodeficiency Virus (HIV), Suid Herpes virus type 1 (SHV-1), Herpes Simplex Virus 1 (HSV-2), Vesicular Stomatitis Virus (VSV), Simian Immunodeficiency Virus (SIV), Feline Calicivirus (FCV), Murine Encephalomyocarditis Virus (MECV), Pseudorabies Virus (PRV), Porcine Parvovirus (PPV), Newcastle Disease Virus (NDV) and Infectious Bursal Disease Virus (IBDV) (Huang et al., 2006; Itokawa et al., 1994; Vollenbroich, Ozel, et al., 1997).

The antimicrobial mechanism of antibiotics is binding of antibiotics to the receptors of specific sites on pathogens so that the normal structure of pathogens is destroyed or certain biosynthesis processes blocked, resulting in bacteriostatic or bactericidal effect. Some studies have focused on exploring the functions of surfactin from the angle of antimicrobial peptides. Surfactin and AMP are both amphiphilic molecules, surfactin is a ring structure, whereas AMPs can be divided into four classes based on their structures: *α* -helix, β-sheet, β-turn loop, boat-shaped. The mechanism of interactions between AMPs and membrane is peptide-lipid interaction which is further categorized into four models: aggregate model, toroidal pore model, barrel-Stave model and carpet model, and most of these models cause death of bacteria through the interactions between lipopeptides by imbalanced inner and outer osmotic pressure. No definite mechanism of the interaction between surfactin and cell membrane has been confirmed thus far; nonetheless, a number of hypotheses have been proposed: 1. detergent-like: this mechanism refers to insertion of a portion of the peptide chain into the middle layer of the cell membrane which causes stronger destabilization of the cell membrane and subsequently results in leak and partitioning of the membrane (Lohner and Epand, 1997; Heeklotz et al., 2004); 2. formation of vesicles: this mechanism can be divided into three parts: (1) insertion of surfactin into the surface of a membrane by hydrophobic interaction between surfactin and the membrane; (2) generation of charge repulsions between negatively charged amino acids and negatively charged lipid headgroups of the surfactin structure and causes membrane bending; (3) membrane destabilization produces micelles which lead to membrane disintegration (Sebastien Buchoux et al., 2008 ; Huang et al., 2009). Studies reported by far suggest that the non-polar end of surfactin may interact with enveloped viruses and generates pores in the membrane which leads to formation of an ion channel and then destroys the entire envelope of these viruses (Vollenbroich et al., 1997). On the other hand, only limited studies relating to the capsid of non-enveloped viruses are available by far; however, Vollenbroich et al. believe surfactin mainly causes damage to viruses by the interactions between its peptide moiety, not the carbon chain of fatty acids, and viral capsid (Vollenbroich et al., 1997). The difference between these two theories is one favors generation of leak while the other proposes formation of pores. In summary, surfactin indeed can cause damage to the membrane which further affects normal physiological function of cells. Studies have reported that antimicrobial peptides have the capacity to destroy Gram-negative bacteria, fungi and protozoa (Powers and Hancock, 2003). Furthermore, other literature also indicates that binding of AMP to the nucleic acids of prokaryotes inhibits protein synthesis (Powers and Hancock, 2003). Comparing with conventional antibiotics, the activity of AMP has several features, such as broad-spectrum, high efficiency, stability and rapid bactericidal activity and capability of interacting with the immune system.

Previous reports have indicated use of antibiotics as additives significantly destroys the intestinal microbial balance of animals and its residues are easily remain in the body which has great impact on the quality of livestock and human health. On the other hand, use of AMPs as feed additives can overcome the above mentioned problems and is the most direct application of AMP in animal husbandry. The AMPs in the intestinal tract of higher animals can inhibit exogenous pathogens while have no killing effects on normal intestinal flora and cells. The ingredients of AMPs are amino acids which can be easily digested and absorbed and thus AMPs can be used as feed additives to replace or partially replace antibiotics currently used in animals and help to reduce the harm of antibiotics to animals. AMPs can replace traditional antibiotics to treat various problematic diseases such as diarrhea in pigs, swine fever, Newcastle disease and cows mastitis; in addition, AMPs will not generate drug-resistant bacteria, are non-toxic and produce no residues and thus has great application potential in treatment and prevention of animal diseases. Using AMPs as feed additives presents numerous advantages which have started to attract people's attention and is very hopeful to become one of the products to replace antibiotics. At present, silkworm liquid yeast preparation AD-peptide is the major product produced domestically and internationally for use as feed additive and most studies in livestock are also performed by using this AMP.

Mold contamination of feed and feed materials is a global problem. To reduce the occurrence of mold contamination, adopt proper measures to minimize the harm of mycotoxins to animal health has become the most important task for international animal husbandry, veterinary medicine and feed technology sector over the years. Initially, people tried to control the water content of the feeds, environment temperature and humidity to reduce the amount of mildew. With the advancement in chemistry and microbiology, chemical preservatives have been developed and the most common ones are organic acids, organic acid salts (esters) and composite mildew-proof agents, but they are all toxic after ingestion. Alternatively, AMP preservatives are proteins which can be easily degraded to amino acids in the GI tract, has only limited side effects in humans and is edible, for example, Nisin has been wildly used in over 50 countries worldwide for food preservation. Non-toxic AMP preservatives made of natural ingredients have slowly replaced traditional chemical preservatives over the years and will play an important role in preservation of the products used in the feed industry and related fields in the future.

Due to increased use of antibiotics in livestock and aquatic animals, drug residues and generation of drug-resistant bacteria have worsened the safety issues of animal foods and posed a threat to human health as well as the development of aquaculture industry; moreover, such problems have caused significant loss in export of livestock products. AMPs are considered to have great application potential because of its broad-spectrum antimicrobial activities, fast bacterial-killing effect, less possibility of generating drug-resistant bacteria and its synergistic effect with traditional antibiotics. Most importantly, AMPs demonstrate great diversity in their sequences and structures, which offers great possibility of imagination and development for designing new drugs and help to produce products with stable performance.

When used as feed additives, AMPs can withstand high temperature during the process of feed pelleting and such feature satisfies the requirements of the procedures for manufacturing feed additives. When used as antibiotics, AMPs exhibit unique mechanisms and have broad-spectrum antimicrobial activities, which help to accelerate the growth of aquatic animals and livestock, maintain health and treat diseases. In addition, AMPs have no toxic side effects, no residuals and do not cause drug resistance, the production cost is low and are not affected by environmental factors such as season or climate change. Industrial production of AMPs containing natural activity from aquatic microorganisms can meet the developmental needs of aquatic and animal husbandry industry as well as help promote the development of green feed additives industry.

More studies have started to use AMPs as feed additives over the past few years and this field is still at the stage of exploration, before mature technology can be developed and large-scale production can become possible, many problems need to be resolved: 1. the natural resources of AMPs are limited and thus chemical synthesis and genetic engineering have become the major means to obtain AMPs; 2. the costs of synthesis is expensive and mass production is still not possible; 3. through genetic engineering, direct expression of the AMP genes in microorganisms may lead to death of the microorganisms or too little of product expression. Most production models employed at present for generation of AMPs mainly produce AMPs by liquid fermentation of engineered bacterial strains and the disadvantages of such method are: 1. the costs for production equipment and liquid fermentation invested at the early stage are very high while the return on investment is slow; 2. using the engineered bacterial stains as feed additives directly without purification may raise the doubt of GMO additives. Therefore, development of AMPs that are natural, have broad spectrum of antimicrobial or bacterial-killing activity, cheap and are non-GMO to replace antibiotics is an important task for medical/pharmaceutical field and development of feed additives. Present invention uses a semi-solid state fermentation method to produce surfactin from a high-yield mutant strain of *Bacillus subtilis* by using soybeans as the fermentation substrate and the resulting soybean powder is used as feed additive after drying and grinding the fermented soybeans.

KR20120126746A describes the production of a feed additive using germinated soybeans fermented by Bacillus subtilis.

### SUMMARY OF THE INVENTION

The present invention provides a method for preparation of feed additives containing surfactin.

Said method comprises the following steps:
Step 1: inoculation of the bacterial culture of the high-yield mutant strain *Bacillus subtilis TH* having the deposit number CGMCC 10270 to a mixture containing 30% mineral salt medium and soybeans and incubate at 30-40°C, humidity 80-90% for semi-solid fermentation for 2-3days to give fermented soybeans; wherein the ratio of the volume of inoculated bacterial culture of the high-yield mutant strain of *Bacillus subtilis* to the volume of soybeans in the mixture is 5:100-10:100.
Step 2: drying the fermented soybeans at 50-60°C for 2-3 hours before grinding and sieving to give soybean powder rich in surfactin (6-7 g per kg); said soybean powder is the feed additive containing surfactin and the amount of surfactin is 6-7 g per kg.

The present invention also provides a method for preparation of feed additives containing surfactin and said feed additive is used as the feed for aquatic animals such as grouper or white shrimp and has the activity to promote the growth rates of aquatic animals and enhance immunity, as well as has antimicrobial, anti-fungal and anti-viral activity.

The present invention also provides a feed for aquaculture, comprising the additive prepared according to the aforementioned method.

The present invention also provides a method for maintaining health of aquatics, comprising administering the effective dosage of the feed to the aquatics in need. Moreover, the present invention is concerned with the inventive feed additive for use in aquaculture as detailed in the accompanying claims.

The feed additive of the present invention is a composition comprising surfactin obtainable by the aforementioned method. The feed additive can be prepared by fermentation, preferably by semi-solid fermentation, of soybeans. More preferably the feed additive, respectively the inventive composition can be obtained by the inventive method as disclosed herein.

### BRIEF DESCRIPTION OF THE DRAWINGS

Fig. 1 shows neutralizing titration results of surfactin at different concentrations in virus-infected cell lines.

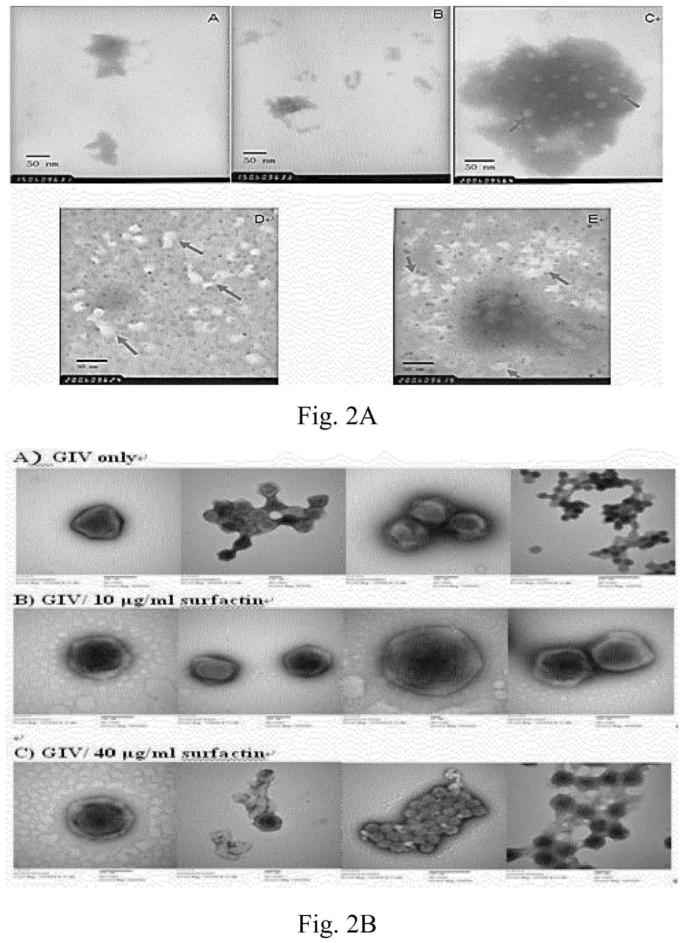

Fig. 2A of Fig. 2 shows the effect of surfactin on morphology of nervous necrosis virus particles, (A) 1%L-15 treatment group; (B) 1% L-15+100 *µ* g/ml surfactin mixed treatment; (C) 1%L-15 nervous necrosis virus-mixed treatment group; (D) 40 *µ* g/ml surfactin+nervous necrosis virus mixed treatment group, the arrow indicates normal viral particles; (E) 100 *µ* g/ml surfactin and nervous necrosis virus mixed treatment group and the arrow indicates abnormal viral particles. Fig. 2B shows the effect of surfactin on morphology of Iridovirus particles, (A) normal Iridovirus group; (B) 10 *µ* g/ml surfactin+ Iridovirus treatment group, the appearance of said virus has changed; (C) 40 *µ* g/ml surfactin+ Iridovirus mixed treatment group, adhesion between surfactin and viruses is observed.
Fig. 3 shows promotion of white shrimp growth by the feed additive containing surfactin.
Fig. 4 shows promotion of grouper growth by feed additive containing surfactin.
Fig. 5 shows the expression of immune genes is increased by the feed additive containing surfactin.
Fig. 6A of Fig. 6 shows the food additive containing surfactin reduces the mortality of grouper infected with nervous necrosis virus. Fig. 6B shows the food additive containing surfactin reduces the mortality rate of grouper infected with Iridovirus.

### DETAILED DESCRIPTION OF THE PREFERRED EMBODIMENT

### Example 1. Preparation of the food additive containing surfactin by semi-solid state fermentation.

This patent utilizes high-yield mutant strain of *Bacillus subtilis* (BCRC No. CGMCC 10270) for semi-solid state fermentation by using soybeans as the substrates and the surface of the fermented soybeans is rich in secondary metabolite-surfactin; the substrate of fermentation can be other cheaper substrates such as rape seed meal, palm oil cake, coconut oil cake, poonac, agricultural byproducts or waste and trash (potato peel, cassava peel and apple peel).

The 10% volume bacterial culture of high-yield mutant strain of *Bacillus subtilis* was inoculated to the mixture of 30% volume mineral salt medium and soybeans and subjected to semi-solid state fermentation for 48 hours under the condition of 30 °C, 80% humidity, followed by drying the fermented soybeans at 55°C for 2-3 hours. After grinding, the soybeans were sieved by a 60-mesh sieve and stored at 4°C, wherein each kilogram of the soybean powder contains 6-7 g of surfactin.

### Example 2. Antimicrobial testing for surfactin

To examine whether surfactin has antimicrobial activity, in vitro susceptibility test was conducted.

Preparation of bacterial culture of Escherichia coli DH5α, Vibrio harveyi, Vibrio alginolyticus, Vibrio anguillarum, Vibrio salmonicida, Aeromonas hydrophila and Staphylococcus epidermidis by inoculation of these bacteria onto LBA (E. coli DH5α) or TSA (+ 1.5%NaCl) and incubation at 37°C for 16 hours. Later, the colonies were scraped off and dissolved in specific medium; the concentration was adjusted to OD₅₄₀ of 1 (1×10⁹ colonies /c.c. culture medium), 100µl of bacterial culture were add to 900µl of LB or TSB(+ 1.5%NaCl) to give a concentration of 5×10⁵ CFU/ml.

### Preparation of fungal cultures

Aspergillus niger was inoculated onto the MEAIII plate and incubated at 25°C for 48 hours, the colonies were scraped off into specific culture medium. Bacterial culture was spread onto the MEAIII plate after serial dilution and the number of colonies was calculated, the original concentration was determined to be 1 × 10⁶ CFU/ml. An aliquot of 500 *µ* l of the bacterial culture was add to 500 *µ* l of MEB before diluting the concentration of bacterial culture to 5 × 10⁵ CFU/ml.

### Susceptibility test

An aliquot of 130µl of the bacterial culture was added to each well of a 96-well plate, the concentration of bacterial culture was 5×10⁵ CFU/ml, 20µl of surfactin at different concentrations prepared by titration were added and incubated at 37°C for 16 hours. A spectrophotometer was used to calculate the concentration of bacterial growth and the obtained data was compared with the original value (OD₅₄₀ =1), the concentration of the experimental group that equals to the original value is used as the minimum inhibiting concentration (MIC). Each group was prepared in triplicate and a control group was included.

The results are shown in Table 1: when compared with the surfactin produced by *Bacillus subtilis* ATCC21332, the MIC of the surfactin produced by the high-yield mutant strain of *Bacillus subtilis* was 96.5 *µ* M.

**Table 1. Comparison of the minimum inhibiting concentration (MIC) (µ M) of surfactin produced by Bacillus subtilis ATCC21332 and the high-yield mutant strain of Bacillus subtilis.**

| | *Bacillus subtilis* ATCC21332 | High-yield mutant strain |
|---|---|---|
| Gram positive | | |
| E. coli | 193 | 96.5 |
| Aeromonas | 128.7 | 96.5 |
| Vibrio anguillarum | 128.7 | 96.5 |
| Vibrio alginolyticus | 128.7 | 96.5 |
| Vibrio harveyi | 128.7 | 96.5 |
| Vibrio salmonicida | 128.7 | 96.5 |

| Gram negative | | |
|---|---|---|
| Staphylococcus epidermidis | 128.7 | 96.5 |
| Fungi | | |
| Aspergillus niger | 193 | 128.7 |

### Example 3. Neutralization test of surfactin and nervous necrosis virus (NNV)

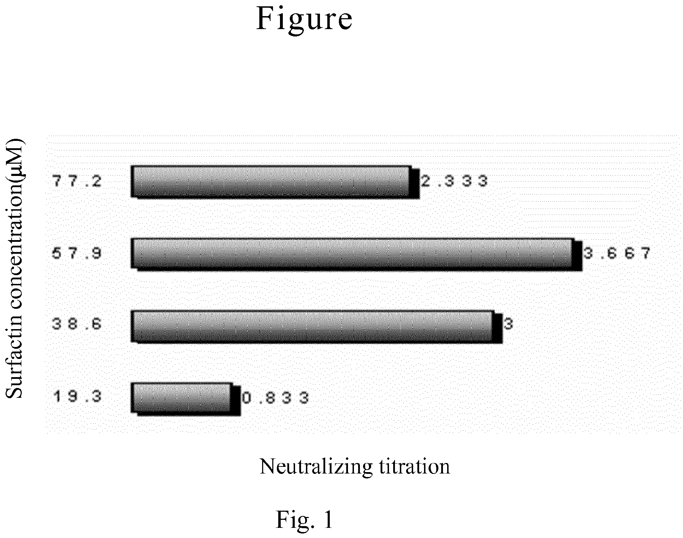

To examine whether surfactin has anti-viral activity, neutralization test was conducted. One day before the test, GF-1 cells were seeded onto a 96-well microplate at the density of 5×10³ cells per well and cultured overnight (16 hours) until 80% confluency before subjected to titration. First, a 10-fold serial dilution, from 10¹ to 10¹⁰, of the test viral culture was performed and then the diluted viral culture was inoculated onto a 96-well plate, 100µl per well. Shake the plate to mix the viral culture, a total of 8 repeats and incubate the plate at 28°C for 90 minutes to allow virus adsorption. The old culture medium was replaced with 200µl of 1% FBS-L15 culture medium and the plate was incubated the 28°C for additional 9 days. The 50% tissue culture infection dose (TCID50) was calculated based on the Reed-Muench Method and is represented by TCID50 for neutralization index (NI=original virus titer/NI titer after surfactin neutralization), the higher the NI titer , the higher the neutralization titer of surfactin. The neutralization reaction was conducted as a 10-fold serial dilution, the neutralization potency is determined as having no neutralization potency when the NI is less than 1 (i.e. the value of Log₁₀NI is less than 1); a NI ranging from 10 to 50 (the value of Log₁₀NI is between 1 and 1.6) indicates the neutralization potency is not significant and is in a doubtful range; while a NI over 50 (the value of Log₁₀NI is greater than 1.7) is determined as having significant neutralization potency (Grace, 1979). The results are shown in Fig.1.

### Example 4. The mechanism of the interactions of surfactin with virus

Surfactin was dissolved in sterilized water and the solution was filtered with a 0.2µm filter membrane, followed by dilution of dissolved surfactin in 1% FBS-L15 to give diluted surfactin solution at the concentrations of 40µg/ml and 100µg/ml. The surfactin treatment group: surfactin and virus in the volume ratio of 1:1 were mixed; control group: 1% FBS-L15 culture medium with NNV culture were mixed and incubated at 28°C for 1 day before examination under a transmission electron microscope (Hitachi H-600). An aliquot of 6µl of the surfactin treatment group was dropped onto a copper mesh to allow precipitation of the virus for about 10 minutes before been stained in 1% phosphotungstic acid (PTA, Sigma) at pH 7.4. After staining, excess mixture was removed with filter paper and incubated at room temperature for 30 minutes, the sample was examined after the copper mesh was air-dried. The voltage used for transmission electron microscope was 75 KV and the exposure time of the film was 4 seconds. The removed negative film was placed on the rack and soaked in the developer D19 for 4 minutes to develop the image. After completion of development, the film was soaked in 0.33% glacial acetic acid to stop the reaction followed by 10 minutes in fixative, finally, excess chemicals were removed by placing the film under running water for 60 minutes before drying. The results are shown in Fig.2.

### Example 5. Growth test of increasing the growth rate of white shrimp by feed additives containing surfactin

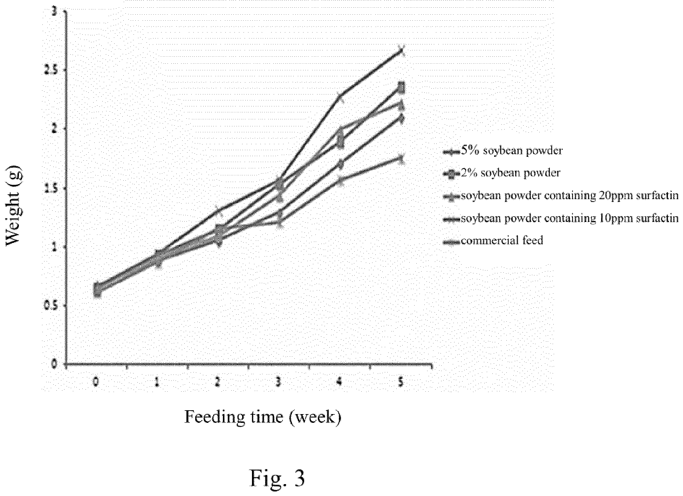

The fermented soybeans and commercial shrimp feed were grounded and then pelletized by using a pellet feed mill after addition of sterilized water, followed by drying at 70°C in an oven for 3 hours to reduce the water content in the feed to 10% and the dried feed was stored at 4°C. The growth test includes 4 feed treatment groups: 1. addition of fermented soybean powder which contains 10 ppm of surfactin, 2. addition of fermented soybean powder which contains 20 ppm of surfactin, 3. addition of water-washed fermented soybean powder 20%, 4, addition of water-washed fermented soybean powder 5%, and control group: commercial feed. Each group includes 25 white shrimps fry and the wet weights were around 0.6 g, the experimental tank was a 30 cm X 24 cm X 60 cm transparent glass cube containing 45 liters of water without temperature control, an air pump was turned on during the entire period of the experiment, and about 1/3 of water was changed each time. The daily feeding amount was 5% of the weights of shrimps and the feeding times were 8 A.M. and 5 P.M. every day, the wet weights were measured and recorded every week for calculation of the growth rate. The results are shown in Fig.3.

### Example 6. Growth test of increasing the growth rate of grouper by feed additive containing surfactin

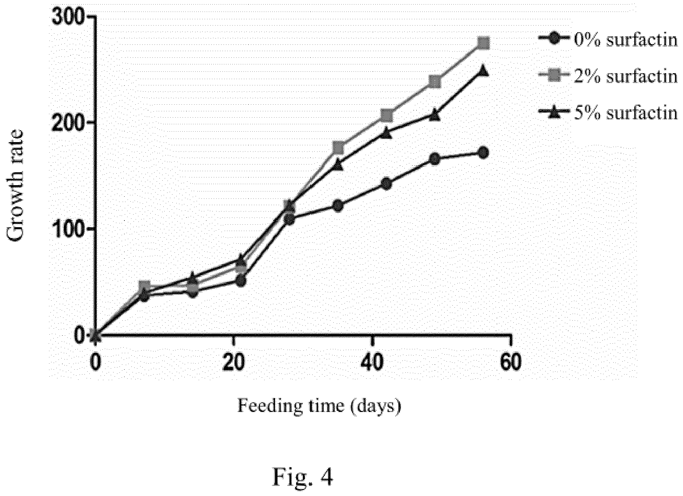

The protein and fat content of the feed were formulated based on the study of Shiau and Lan (1996), the formula of the test feed was common feed with 47% crude protein and 10% fat. Red fish powder, corn gluten protein, wheat gluten protein and squid powder were ground through a grinder and sieved (35 mesh screen) to increase viscosity of the feed. All feeds were pelletized by a pellet feed mill before separation and drying and 2-5% of surfactin was then added before storing at -20°C to ensure feed quality. The results are shown in Fig. 4.

### Example 7. Feed additives containing surfactin can induce innate immune gene expression in the animals

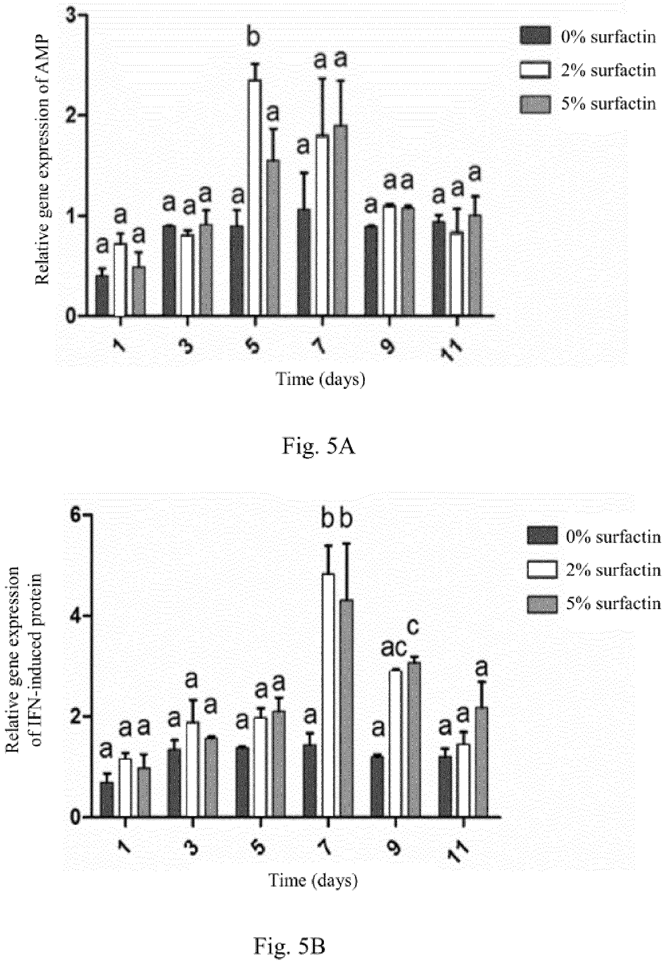

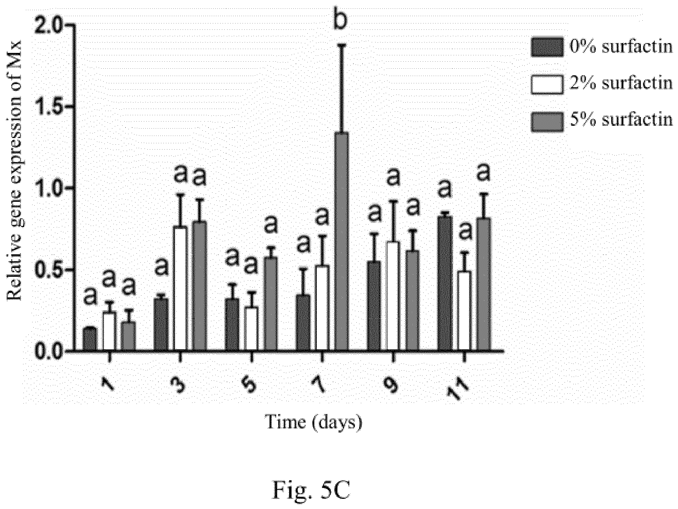

Continuous feeding of grouper with feeds containing 0%, 2% and 5% surfactin can induce expression of the immune genes in grouper including AMP (Antimicrobial peptide), MX (MX protein is a GTPase induced by interferon) and INF-inducted protein. The results are shown in Fig. 5.

### Example 8. Feed additive containing surfactin increases disease resistance of animals

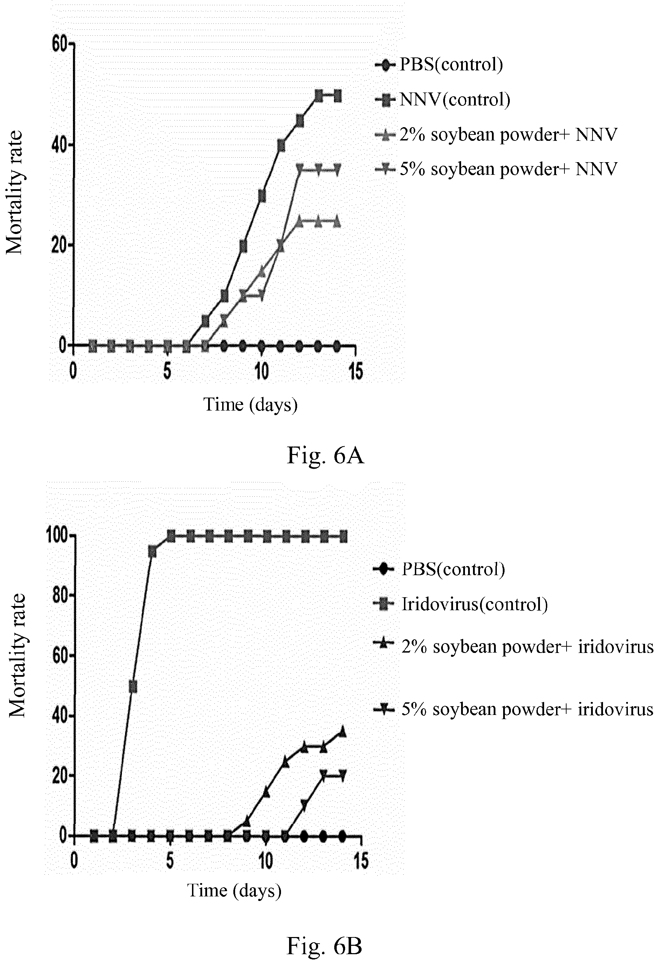

Change in mortality rate in groupers infected with either NNV or Iridovirus after giving the feed containing surfactin for 7 consecutive days to orange spotted grouper fry. The experiment was divided into 4 groups: 1. negative control group: feed with common feed + PBS (phosphate-buffered saline) injection; 2. positive control group: fee with common feed+ virus injection; 3. study group: feed with the feed containing 2% surfactin + virus injection; 4. study group: feed with the feed containing 5% surfactin + virus injection. The results are shown in Fig. 6.

The surfactin produced by the method of present invention disclosed in the abovementioned embodiments can be used in aquaculture feeds and be adapted to the changes of water environment and numerous pathogens; in addition, said surfactin is acid(base)-resistant, heat-resistant, has broad-spectrum antimicrobial activity (Gram-positive and negative, fungi, parasites, viruses), does not cause drug resistance and can be bio-degraded, said surfactin is environmental-friendly and a green feed additive without the problem of drug residues. It is very suitable for use as a new kind of antibiotic and anti-disease feed additive.

The method of the invention uses semi-solid state fermentation and dry and grind the substrate directly after fermentation to give the feed additive which can replace common antibiotics. The production process of this method is simple and the production cost is cheap.

## Claims

1. A method for preparation of feed additives containing surfactin, said method comprising the following steps:
A. inoculation of the bacterial culture of the high-yield mutant strain *Bacillus subtilis TH* having the deposit number CGMCC 10270 to a mixture containing 30% volume mineral salt medium and soybeans for semi-solid fermentation to give fermented soybeans;
B. drying the fermented soybeans before grinding and sieving to give the soybean powder; said soybean powder being the feed additive containing surfactin.

2. The method of claim 1, wherein the volume ratio of inoculated bacterial culture of the high-yield mutant strain of Bacillus subtilis to soybeans in the mixture is 5:100-10:100.

3. The method of claim 1, wherein the semi-solid state fermentation of step A refers to incubation for 2-3 days at 30-40°C and 80-90% humidity.

4. The method of claim 1, wherein the drying temperature of step B refers to drying at 50-60°C for 2-3hours.

5. The method of claim 1, wherein the soybean powder of step B contains 6-7 g/kg of surfactin.

6. Feed additive obtainable by a method according to any one of claims 1 to 5.

7. Composition comprising surfactin for use in aquaculture, wherein the composition comprising surfactin is obtainable by a method according to any one of claims 1 to 5.

8. The composition for use according to claim 7, wherein the aquatic organisms are fish, preferably grouper, and/or shrimp, preferably white shrimp.

9. A composition comprising surfactin for use in a method to promote the growth rate of aquatic organisms, wherein the composition comprising surfactin is obtainable by a method according to any one of claims 1 to 5.

10. A composition comprising surfactin for use in a method to increase the immunity of aquatic organisms, wherein the composition comprising surfactin is obtainable by a method according to any one of claims 1 to 5.

11. A composition comprising surfactin for use in a method to prevent bacterial, fungal and/or viral infections, preferably in fish and/or shrimps, wherein the composition comprising surfactin is obtainable by a method according to any one of claims 1 to 5.

12. A feed for aquaculture, comprising the feed additive obtainable by the method according to any one of claims 1 to 5.

13. Use of a feed additive comprising surfactin, wherein the feed additive is prepared by the method according to any one of claims 1 to 5, as a feed for aquaculture.

## Patentansprüche

1. Verfahren zur Herstellung von Surfactin enthaltenden Futtermittelzusätzen, wobei das Verfahren die folgenden Schritte umfasst:
A. Beimpfen einer Mischung, die 30 Volumen-% Mineralsalzmedium und Sojabohnen enthält, mit der Bakterienkultur des hoch ertragreichen Mutantenstammes *Bacillus subtilis* TH, der die Hinterlegungsnummer CGMCC 10270 aufweist, zur halbfesten Fermentation, um fermentierte Sojabohnen zu ergeben;
B. Trocknen der fermentierten Sojabohnen vor einem Mahlen und Sieben, um das Sojabohnenpulver zu ergeben; wobei das Sojabohnenpulver der Surfactin enthaltende Futtermittelzusatz ist.

2. Verfahren nach Anspruch 1, wobei das Volumenverhältnis von inokulierter Bakterienkultur des hoch ertragreichen Mutantenstammes von *Bacillus subtilis* zu Sojabohnen in der Mischung 5: 100 - 10: 100 beträgt.

3. Verfahren nach Anspruch 1, wobei sich die Halbfeststofffermentation von Schritt A auf eine Inkubation für 2 bis 3 Tage bei 30 bis 40 ° C und 80 bis 90 % Feuchtigkeit bezieht.

4. Verfahren nach Anspruch 1, wobei sich die Trocknungstemperatur von Schritt B auf ein Trocknen bei 50 bis 60 °C für 2 bis 3 Stunden bezieht.

5. Verfahren nach Anspruch 1, wobei das Sojabohnenpulver von Schritt B 6 bis 7 g/kg Surfactin enthält.

6. Futtermittelzusatz, erhältlich durch ein Verfahren nach einem der Ansprüche 1 bis 5.

7. Surfactin umfassende Zusammensetzung zur Verwendung in der Aquakultur, wobei die Surfactin umfassende Zusammensetzung durch ein Verfahren nach einem der Ansprüche 1 bis 5 erhältlich ist.

8. Zusammensetzung zur Verwendung nach Anspruch 7, wobei die Wasserorganismen Fisch, vorzugsweise Zackenbarsch und / oder Garnele, vorzugsweise weiße Garnele, sind.

9. Surfactin umfassende Zusammensetzung zur Verwendung in einem Verfahren zum Fördern der Wachstumsrate von Wasserorganismen, wobei die Surfactin umfassende Zusammensetzung durch ein Verfahren nach einem der Ansprüche 1 bis 5 erhältlich ist.

10. Surfactin umfassende Zusammensetzung zur Verwendung in einem Verfahren zum Erhöhen der Immunität von Wasserorganismen, wobei die Surfactin umfassende Zusammensetzung durch ein Verfahren nach einem der Ansprüche 1 bis 5 erhältlich ist.

11. Surfactin umfassende Zusammensetzung zur Verwendung in einem Verfahren zum Verhindern von bakteriellen, pilzlichen und / oder viralen Infektionen, vorzugsweise in Fischen und / oder Garnelen, wobei die Surfactin umfassende Zusammensetzung durch ein Verfahren nach einem der Ansprüche 1 bis 5 erhältlich ist.

12. Futtermittel für die Aquakultur, umfassend den durch das Verfahren nach einem der Ansprüche 1 bis 5 erhältlichen Futtermittelzusatz.

13. Verwendung eines Surfactin umfassenden Futtermittelzusatzes, wobei der Futtermittelzusatz durch das Verfahren nach einem der Ansprüche 1 bis 5 hergestellt wird, als ein Futtermittel für die Aquakultur.

## Revendications

1. Procédé de préparation d'additifs alimentaires contenant de la surfactine, ledit procédé comprenant les étapes suivantes :
A. inoculation de la culture bactérienne de la souche mutante à rendement élevée *Bacillus subtilis TH* ayant le numéro d'accès CGMCC 10270 à un mélange contenant un milieu de sel minéral à 30 % en volume et du soja pour la fermentation semi-solide afin d'obtenir du soja fermenté ;
B. séchage du soja fermenté avant le broyage et le tamisage pour obtenir la poudre de soja ; ladite poudre de soja étant l'additif alimentaire contenant la surfactine.

2. Procédé selon la revendication 1, dans lequel le rapport en volume de la culture bactérienne inoculée de la souche mutante à rendement élevé de *Bacillus subtilis* sur le soja dans le mélange est de 5/100 à 10/100.

3. Procédé selon la revendication 1, dans lequel l'état de fermentation semi-solide de l'étape A fait référence à une incubation pendant 2 à 3 jours, à 30 à 40 °C et une humidité de 80 à 90 %.

4. Procédé selon la revendication 1, dans lequel la température de séchage de l'étape B fait référence à un séchage à 50 à 60 °C pendant 2 à 3 heures.

5. Procédé selon la revendication 1, dans lequel la poudre de soja de l'étape B contient 6 à 7 g/kg de surfactine.

6. Additif alimentaire pouvant être obtenu par un procédé selon l'une quelconque des revendications 1 à 5.

7. Composition comprenant de la surfactine pour son utilisation en aquaculture, dans laquelle la composition comprenant de la surfactine peut être obtenue par un procédé selon l'une quelconque des revendications 1 à 5.

8. Composition pour son utilisation selon la revendication 7, dans laquelle les organismes aquatiques sont des poissons, de préférence le mérou, et/ou des crevettes, de préférence la crevette blanche.

9. Composition comprenant de la surfactine pour son utilisation dans un procédé de promotion du taux de croissance d'organismes aquatiques, dans laquelle la composition comprenant la surfactine peut être obtenue par un procédé selon l'une quelconque des revendications 1 à 5.

10. Composition comprenant de la surfactine pour son utilisation dans un procédé d'augmentation de l'immunité d'organismes aquatiques, dans laquelle la composition comprenant la surfactine peut être obtenue par un procédé selon l'une quelconque des revendications 1 à 5.

11. Composition comprenant de la surfactine pour son utilisation dans un procédé de prévention d'infections bactériennes, fongiques et/ou virales, de préférence chez les poissons et/ou les crevettes, dans laquelle la composition comprenant la surfactine peut être obtenue par un procédé selon l'une quelconque des revendications 1 à 5.

12. Aliment pour l'aquaculture, comprenant l'additif alimentaire pouvant être obtenu par le procédé selon l'une quelconque des revendications 1 à 5.

13. Utilisation d'un additif alimentaire comprenant de la surfactine, dans laquelle l'additif alimentaire est préparé par le procédé selon l'une quelconque des revendications 1 à 5, en tant qu'aliment pour l'aquaculture.
